# EUROPEAN PATENT APPLICATION

(11) **EP 4 286 361 A1**
(43) Date of publication of application: **06.12.2023**
(21) Application number: 22745585.4
(22) Date of filing: 13.01.2022
(51) Int. Cl.: C07C 67/08, C07C 69/54, C08G 65/48, C08F 20/20, C08F 20/30

(54) **METHOD FOR PRODUCING (METH)ACYRLIC ACID ESTER COMPOUND**

(30) Priority: 27.01.2021 JP 2021010870
(71) Applicant: MITSUBISHI GAS CHEMICAL COMPANY, INC., Chiyoda-ku Tokyo 100-8324 (JP)
(72) Inventor: AOYAGI Naoto, Tokyo 125-0051 (JP); UOTANI Takuya, Tokyo 125-0051 (JP)
(74) Representative: Hoffmann Eitle
(86) International application number: PCT/JP2022/000838
(87) International publication number: WO 2022/163360

(57) **Abstract**

To provide a method for producing a (meth)acrylic acid ester compound, wherein the method enables the esterification at a high introduction rate and collecting the obtained ester compound efficiently. A method for producing a (meth)acrylic acid ester compound, comprising: reacting a polymer having a structure represented by formula (1) with (meth)acrylic acid anhydride in the presence of at least one of potassium carbonate, rubidium carbonate, and cesium carbonate. In formula (1), R¹, R², R³, R⁴, and R⁵ are each independently selected from a hydrogen atom and an alkyl group, and at least one of R¹, R², R³, R⁴, and R⁵ is selected from a single bond, -O-*, -S-*, -S(= O)-*, -S(=O)₂-*, and an alkylene group-*; and X is a hydrogen atom, at least some of which react with (meth)acrylic acid anhydride to be (meth)acrylic groups.

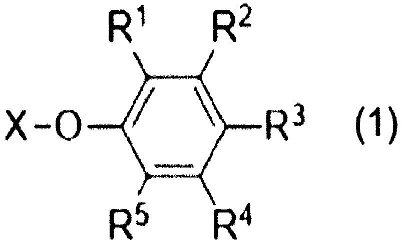

## Description

### [Technical Field]

The present invention relates to a method for producing a (meth)acrylic acid ester compound.

### [Background Art]

The production of ester compounds by esterifying phenolic compounds has been examined until now.

For example, Patent Literature 1 discloses a method for producing a phenyl ester, comprising: esterifying a phenol and a carboxylic acid represented by the following general formula (I): 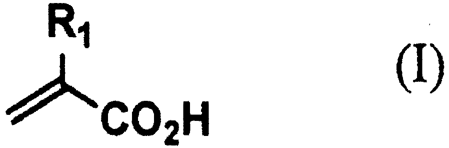 wherein R₁ represents hydrogen or a methyl group,
with an acid catalyst in a solvent forming an azeotrope with water, wherein, in the reaction system of the esterification reaction, boric acid and a 2,2-dialkyl malonic acid represented by the following general formula (II): 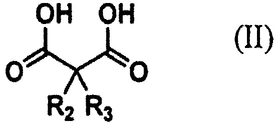 wherein R₂ or R₃ represents a linear or branched alkyl group having 2 to 10 carbon atoms,
are each added to the carboxylic acid represented by general formula (I) at 2 to 50% by mol.

Patent Literature 2 discloses a curable composition, comprising: a capped poly(arylene ether) produced by the reaction of an uncapped poly(arylene ether) with an anhydride capping agent, and an olefinic unsaturated monomer, wherein the water absorption of the cured composition at 85°C and a relative humidity of 85% after 7 days is less than 1% by weight. Here, it is disclosed that the uncapped poly(arylene ether) and the anhydride capping agent are reacted in the presence of a capping catalyst containing 4-dialkylaminopyridine.

Furthermore, Patent Literature 3 discloses that in the presence of sodium acetate, a predetermined polyphenylene oxide oligomer is reacted with 2-methacrylic acid anhydride (methacrylic anhydride) to obtain a polyphenylene oxide oligomer in which the end groups are functionalized.

### [Citation List]

### [Patent Literature]

[Patent Literature 1]
   Japanese Patent Laid-Open No. 2011-105667
[Patent Literature 2]
   Japanese Translation of PCT International Application Publication No. 2007-507592
[Patent Literature 3]
   Japanese Patent Laid-Open No. 2019-210451

### [Summary of Invention]

### [Technical Problem]

As described above, methods for esterifying phenolic compounds have been disclosed. However, on the examination of the present inventor, when the esterification of an oligo phenylene ether having hydroxy groups at both ends was attempted in accordance with the description of Example in Patent Literature 1, the ester introduction rate was very low. When the esterification of an oligo phenylene ether having hydroxy groups at both ends was attempted using 4-dimethylaminopyridine (DMAP) as a catalyst in accordance with the description of Example in Patent Literature 2, it was difficult to separate the catalyst from the product, and a large amount of by-products also remained. DMAP is originally expensive, and also has a problem with cost. Meanwhile, it was found that when sodium acetate, used in Example in Patent Literature 3, was used as a catalyst, the introduction of (meth)acrylic groups was difficult without using an excessive amount of (meth)acrylic acid anhydride.

The present invention solves such problems, and an object thereof is to provide a method for producing a (meth)acrylic acid ester compound, wherein the method involves obtaining an ester compound produced by esterifying a phenolic compound, and enables the esterification at a high introduction rate and collecting the obtained ester compound efficiently.

### [Solution to Problem]

In the above-mentioned problem, it has been found that the above-mentioned problem can be solved by the esterification using (meth)acrylic acid anhydride in the presence of a predetermined basic catalyst. The above-mentioned problem has been specifically solved by the following means.
<1> A method for producing a (meth)acrylic acid ester compound, comprising: reacting a polymer having a structure represented by formula (1) with (meth)acrylic acid anhydride in the presence of at least one of potassium carbonate, rubidium carbonate, and cesium carbonate 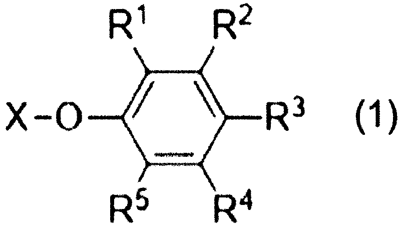 wherein R¹, R², R³, R⁴, and R⁵ are each independently selected from a hydrogen atom and an alkyl group, and at least one of R¹, R², R³, R⁴, and R⁵ is selected from a single bond, -O-*,-S-*, -S(= O)-*, - S(=O)₂-*, and an alkylene group-*, where * is an attachment position to any other site; and X is a hydrogen atom, at least some of which react with (meth)acrylic acid anhydride to be (meth)acrylic groups.
<2> The method for producing a (meth)acrylic acid ester compound according to <1>, wherein a proportion of a polymer having the structure represented by formula (1) wherein X is a hydrogen atom, after reaction with (meth)acrylic acid anhydride, in the polymer having the structure represented by formula (1) is 10% by mol or less.
<3> The method for producing a (meth)acrylic acid ester compound according to <1> or <2>, wherein the polymer having the structure represented by formula (1) is further reacted with an acyl compound, and some of X after the reaction are acyl groups.
<4> The method for producing a (meth)acrylic acid ester compound according to any one of <1> to <3>, wherein a number average molecular weight of the polymer having the structure represented by formula (1) is 1,000 to 10,000.
<5> The method for producing a (meth)acrylic acid ester compound according to any one of <1> to <4>, wherein a hydroxyl value, which is a mass of the polymer having the structure represented by formula (1) per 1 mol of hydroxy groups of the polymer, is 100 to 5,000 g/mol.
<6> The method for producing a (meth)acrylic acid ester compound according to any one of <1> to <5>, wherein a total of 1.0 to 10.0 mol of at least one of potassium carbonate, rubidium carbonate, and cesium carbonate, and 1.0 to 10.0 mol of (meth)acrylic acid anhydride are used per 1 mol of the hydroxy groups of the polymer having the structure represented by formula (1).
<7> The method for producing a (meth)acrylic acid ester compound according to any one of <1> to <6>, wherein the polymer having the structure represented by formula (1) comprises a polymer represented by formula (2) or formula (3) 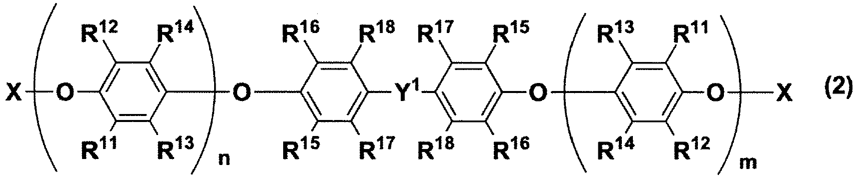 wherein R¹¹ to R¹⁸ are each independently selected from a hydrogen atom and an alkyl group; Y¹ is a single bond, -O-, -S-, -S(=O)-, - S(=O)₂-, -C(=O)-, or -C(R⁹)(R¹⁰)-, where R⁹ and R¹⁰ are each independently a hydrogen atom, an alkyl group, an alkynyl group, a hydroxy group, an amino group, an aryl group, or a heterocyclic group, and R⁹ and R¹⁰ may be bound to each other to form a ring structure; X is a hydrogen atom, at least some of which react with (meth)acrylic acid anhydride to be (meth)acrylic groups; and n is an integer of 0 or more, m is an integer of 0 or more, and m + n is an integer of 1 or more, 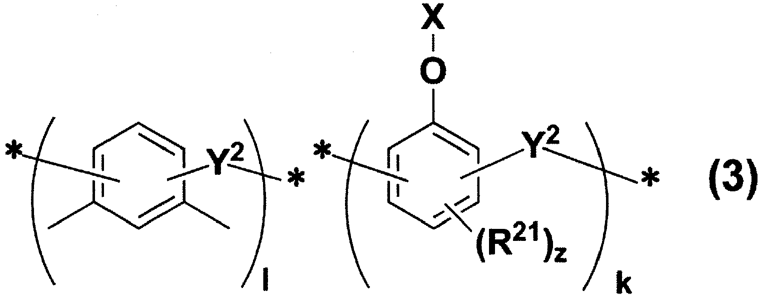 wherein R²¹ is an alkyl group, a hydroxy group, or an aryl group; Y² is -CH₂-, -CH₂O-, or -CH₂OCH₂-; X is a hydrogen atom, at least some of which react with (meth)acrylic acid anhydride to be (meth)acrylic groups; 1 is an integer of 1 or more; k is an integer of 2 or more;, z is an integer of 0 to 3; and * is an attachment position to any other constituent unit or an end group.
<8> The method for producing a (meth)acrylic acid ester compound according to any one of <1> to <7>, wherein the reaction is performed in the presence of at least one of an aromatic hydrocarbon solvent and an ether solvent.

### [Advantageous Effects of Invention]

The present invention can provide a method for producing a (meth)acrylic acid ester compound, wherein the method involves obtaining an ester compound by esterifying a phenolic compound, and enables the esterification at a high introduction rate and collecting the obtained ester compound efficiently.

### [Brief Description of Drawings]

[Figure 1] Figure 1 shows the ¹H NMR spectrum of a raw material resin (SA90) compound.
[Figure 2] Figure 2 shows the ¹H NMR spectrum of a (meth)acrylic acid ester compound obtained in Example 5.
[Figure 3] Figure 3 shows the ¹H NMR spectrum of a (meth) acrylic acid ester compound obtained in Comparative Example 1.

### [Description of Embodiments]

Hereinafter, aspects for implementing the present invention (hereinafter referred to merely as "the present embodiments") will be described in detail. The following present embodiments are illustration for describing the present invention, and the present invention is not limited only to the present embodiment.

The word "to" used herein means that numerical values described at the left and right thereof are inclusive as a lower limit value and an upper limit value.

Various physical property values and characteristic values herein are physical property values and characteristic values at 23°C unless otherwise specified.

In the expression of a group (atomic group) used herein, the expression not mentioning whether the group is substituted or unsubstituted includes a group (atomic group) having no substituent and a group (atomic group) having a substituent(s). For example, an "alkyl group" includes not only an alkyl group not having a substituent (unsubstituted alkyl group) but also an alkyl group having a substituent(s) (substituted alkyl group). When the expression not mentioning whether the group is substituted or unsubstituted is used herein, the unsubstituted group is more preferable.

"(Meth)acryl" used herein represents both or either of acryl and methacryl. In the present invention, methacryl is preferable.

If a measuring method and the like based on a standard shown herein vary depending on the year, the measuring method and the like are based on the standard as of the application date unless otherwise specified.

A method for producing a (meth)acrylic acid ester compound of the present embodiment (hereinafter occasionally referred to merely as a "production method of the present embodiment") is characterized by comprising reacting a polymer having a structure represented by formula (1) with (meth)acrylic acid anhydride in the presence of at least one of potassium carbonate, rubidium carbonate, and cesium carbonate. Such a configuration enables the esterification at a high introduction rate and collecting the obtained (meth)acrylic acid ester compound efficiently. For example, in the production method of the present embodiment, almost all of the surplus reagent and the by-product can be removed by one filtration after the reaction, and the (meth)acrylic acid ester compound that is a target substance can be collected at a high yield.

In the present embodiment, the esterification reaction is advanced in the presence of at least one of potassium carbonate, rubidium carbonate and cesium carbonate. That is, it is assumed that potassium ions, rubidium ions, or cesium ions convert phenolic hydroxy group (OX) sites in formula (1) into O⁻, and the reaction proceeds. It is estimated that since especially potassium ions, rubidium ions, and cesium ions have large sizes as cations, phenoxy anions are easily made free, and the nucleophilic attacking properties on an acylating agent can be increased. Meanwhile, sodium ions and lithium ions are less able to ionize OH into O⁻, it is assumed that even the ionization of OH reduces the reactiveness with the acylating agent due to strong ion bonding force between O⁻ and sodium ions or lithium ions. Since potassium hydrogen carbonate or the like has a smaller ionic radius, is less basic and less able to convert OH into O⁻ under the influence of hydrogen (H) than potassium carbonate, it is assumed that potassium hydrogen carbonate or the like is low reactive. In the present embodiment, a carbonate is used. It is assumed that when a more strongly basic catalyst is adopted than a carbonate, the catalyst attacks methacrylic acid anhydride. For example, potassium hydroxide or cesium hydroxide decomposes methacrylic acid anhydride into methacrylic acid. In the present embodiment, the carbonate is selected so as to activate the phenolic hydroxy group (OX) site that formula (1) has preferentially. Furthermore, the carbonate is low-priced, and the industrial utility value is high.

Furthermore, the (meth)acrylic acid ester compound obtained by the production method of the present embodiment can have performance equivalent to conventional low dielectric resins. Furthermore, a resin also having high glass transition temperature is obtained, and a material excellent in heat resistance is obtained.

Hereinafter, details about the production method of the present embodiment will be described.

In the production method of the present embodiment, the polymer having the structure represented by formula (1) is reacted with (meth)acrylic acid anhydride for esterification reaction. Consequently, a (meth)acrylic acid ester compound in which a (meth)acrylic group is introduced into a phenolic hydroxy group that the polymer having the structure represented by formula (1) has, namely an X site in formula (1), is obtained.

In the production method of the present embodiment, the polymer having the structure represented by formula (1) is used as a raw material. The use of such a resin enables producing a thermosetting resin excellent in low dielectric properties and heat resistance. 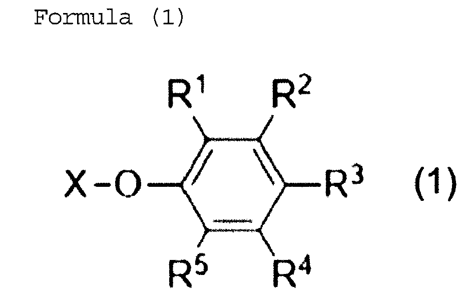 wherein R¹, R², R³, R⁴, and R⁵ are each independently selected from a hydrogen atom and an alkyl group, and at least one of R¹, R², R³, R⁴, and R⁵ is selected from a single bond, -O-*, -S-*, -S(= O) -*, - S(=O)₂-*, and an alkylene group-*, where * is an attachment position to any other site; and X is a hydrogen atom, at least some of which react with (meth)acrylic acid anhydride to be (meth)acrylic groups.

R¹, R², R³, R⁴, and R⁵ are each independently selected from a hydrogen atom and an alkyl group. The alkyl group is preferably an alkyl group having 1 to 10 carbon atoms, and more preferably an alkyl group having 1 to 5 carbon atoms. Although the alkyl group may be any of linear, branched, and cyclic alkyl groups, the alkyl group is preferably linear or branched, and further preferably linear. Although the alkyl group may have a substituent(s), or may not have a substituent, it is preferable that the alkyl group do not have a substituent. If the alkyl group has a substituent (s), as the substituent, a halogen atom, an alkenyl group, an alkynyl group, and an aryl group are exemplified. Specifically, the alkyl group is preferably a methyl group, an ethyl group, or a propyl group, and further preferably a methyl group.

Among the single bond, -O-*, -S-*, -S(=O)-*, -S(=O)₂-* or alkylene group-*, -O-*, -S-*, -S(=O)-*, -S(=O)₂-*, or an alkylene group-* is preferable, and -O-* is more preferable.

In the present embodiment, it is preferable that two or three of R¹, R², R³, R⁴, and R⁵ be hydrogen atoms, two or three thereof be alkyl groups (preferably methyl groups), and the remainder be a single bond, -O-*, -S-*, -S(= O)-*, -S(=O)₂-*, or an alkylene group-* (preferably -O-*). It is more preferable that R² and R⁴ be hydrogen atoms, R¹ and R⁵ be alkyl groups (preferably methyl groups), and R³ be a single bond, -O-*, -S-*, -S(=O)-*, - S(=O)₂-*, or an alkylene group-*.

In formula (1), * indicates an attachment position to any other site, and is usually bound to the main chain of the polymer. However, the structure represented by formula (1) may be bound to a side chain of the polymer.

In the polymer having a structure represented by formula (1) (raw material), X is a hydrogen atom, at least some of which react with (meth)acrylic acid anhydride to be (meth)acrylic groups. In the present embodiment, a proportion of a polymer having the structure represented by formula (1) wherein X is a hydrogen atom, after reaction with (meth)acrylic acid anhydride, in the polymer having the structure represented by formula (1) is preferably 150 by mol or less, more preferably 10% by mol or less, further preferably 7% by mol or less, and still more preferably 3% by mol or less. The lower limit value of the proportion of a polymer having the structure represented by formula (1) wherein X is a hydrogen atom is preferably 0% by mol or more. As described below in detail, some of X may be reacted with an acyl compound or the like to be acyl groups or the like.

As long as the polymer having the structure represented by formula (1) has a structure represented by formula (1), the polymer is not particularly limited. The polymer may have the structure represented by formula (1) at an end of the polymer, or may have the structure at a portion other than the ends of polymer. Examples of one embodiment of the polymer having the structure represented by formula (1) include a polymer having the structure represented by formula (1) at at least one end (preferably both ends). As one embodiment of the polymer having the structure represented by formula (1), a polyphenylene ether compound having the structure represented by formula (1) is exemplified.

Specifically, the polymer having the structure represented by formula (1) is preferably a polymer represented by the following formula (2) or formula (3). 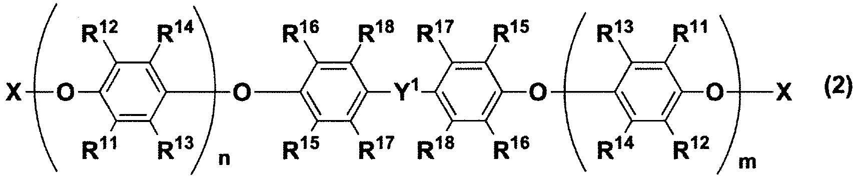 wherein R¹¹ to R¹⁸ are each independently selected from a hydrogen atom and an alkyl group; Y¹ is a single bond, -O-, -S-, -S(=O)-, - S(=O)₂-, -C(=O)-, or -C(R⁹)(R¹⁰)-, where R⁹ and R¹⁰ are each independently a hydrogen atom, an alkyl group, an alkynyl group, a hydroxy group, an amino group, an aryl group, or a heterocyclic group, and R⁹ and R¹⁰ may be bound to each other to form a ring structure; X is a hydrogen atom, at least some of which react with (meth)acrylic acid anhydride to be (meth)acrylic groups; and n is an integer of 0 or more, m is an integer of 0 or more, and m + n is an integer of 1 or more.

R¹¹ to R¹⁸ are each independently selected from a hydrogen atom and an alkyl group. The alkyl group is preferably an alkyl group having 1 to 10 carbon atoms, and more preferably an alkyl group having 1 to 5 carbon atoms. The alkyl group may be any of linear, branched, and cyclic alkyl groups, the alkyl group is preferably linear or branched, and further preferably linear. Although the alkyl group may have a substituent(s), or may not have a substituent, it is preferable that the alkyl group do not have a substituent. If the alkyl group has a substituent (s), as the substituent, a halogen atom, an alkenyl group, an alkynyl group, and an aryl group are exemplified. Specifically, the alkyl group is preferably a methyl group, an ethyl group, or a propyl group, and further preferably a methyl group.

It is preferable that one to three of R¹¹, R¹², R¹³, and R¹⁴ be hydrogen atoms, and the remainder be an alkyl group (preferably a methyl group) . It is more preferable that R¹³ and R¹⁴ be hydrogen atoms, and R¹¹ and R¹² be alkyl groups (preferably methyl groups) .

It is preferable that one to three of R¹⁵, R¹⁶, R¹⁷, and R¹⁸ be hydrogen atoms, the remainder be an alkyl group(s) (preferably a methyl group (s)) . It is more preferable that one or two thereof be hydrogen atoms, and the remainder be an alkyl group(s) .

Y¹ is a single bond, -O-, -S-, -S(=O)-, -S(=O)₂-, -C(=O)-, or -C(R⁹)(R¹⁰)-, and is preferably a single bond, -O-, or - C(R⁹)(R¹⁰)-, and more preferably a single bond or -C(R⁹)(R¹⁰)-.

R⁹ and R¹⁰ are each independently a hydrogen atom, an alkyl group, an alkynyl group, a hydroxy group, an amino group, an aryl group, or a heterocyclic group, and R⁹ and R¹⁰ may be bound to each other to form a ring structure. Although the alkyl group, the alkynyl group, the aryl group, and the heterocyclic group may have substituents, or may not have substituents, it is preferable that the alkyl group, the alkynyl group, the aryl group, and the heterocyclic group do not have substituents. As the substituents, a halogen atom, an alkenyl group, an alkynyl group and an aryl group are exemplified.

It is preferable that R⁹ and R¹⁰ be each independently a hydrogen atom, an alkyl group having 1 to 10 carbon atoms, an alkynyl group having 2 to 10 carbon atoms, a hydroxy group, an amino group, an aryl group having 6 to 12 carbon atoms, or a 5- or 6-membered heterocyclic group. It is more preferable that R⁹ and R¹⁰ be a hydrogen atom, an alkyl group having 1 to 5 carbon atoms, or a hydroxy group. It is further preferable that R⁹ and R¹⁰ be a hydrogen atom or a methyl group. It is still more preferable that R⁹ and R¹⁰ be a methyl group.

X is synonymous with X in formula (1).

n is an integer of 0 or more, preferably an integer of 1 or more, and more preferably an integer of 5 or more. n is preferably an integer of 50 or less, and more preferably an integer of 20 or less.

m is an integer of 0 or more, preferably an integer of 1 or more, and more preferably an integer of 5 or more. m is preferably an integer of 50 or less, and more preferably an integer of 20 or less.

m + n is an integer of 1 or more, preferably an integer of 10 or more, and more preferably an integer of 11 or more. m + n is preferably an integer of 100 or less, and more preferably an integer of 30 or less. 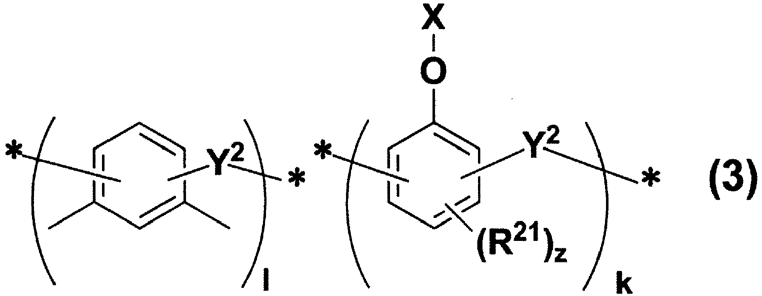 wherein R²¹ is an alkyl group, a hydroxy group, or an aryl group, Y² is -CH₂-, -CH₂O-, or -CH₂OCH₂-, X is a hydrogen atom, at least some of which react with (meth)acrylic acid anhydride to be (meth)acrylic groups, 1 is an integer of 1 or more, k is an integer of 2 or more, z is an integer of 0 to 3, and * is an attachment position to any other constituent unit or an end group.

R²¹ is an alkyl group, a hydroxy group, or an aryl group, and is preferably an alkyl group.

The alkyl group is preferably an alkyl group having 1 to 15 carbon atoms, more preferably an alkyl group having 2 to 10 carbon atoms, and more preferably an alkyl group having 3 to 7 carbon atoms. Although the alkyl group may be any of linear, branched, and cyclic alkyl groups, the alkyl group is preferably linear or branched, and further preferably branched. Although the alkyl group may have a substituent(s), or may not have a substituent, it is preferable that the alkyl group do not have a substituent. If the alkyl group has a substituent(s), as the substituent, a halogen atom, an alkenyl group, an alkynyl group, and an aryl group are exemplified. Specifically, the alkyl group is preferably a methyl group, an ethyl group, or a butyl group, and further preferably a t-butyl group.

The aryl group is preferably a phenyl group.

Y² is -CH₂-, -CH₂O-, or -CH₂OCH₂-, and is preferably -CH₂-.

X is synonymous with X in formula (1).

1 is an integer of 1 or more, preferably an integer of 4 or more, and more preferably an integer of 5 or more. 1 is preferably an integer of 50 or less, and more preferably an integer of 20 or less.

k is an integer of 2 or more, preferably an integer of 3 or more, and more preferably an integer of 6 or more. k is preferably an integer of 50 or less, and more preferably an integer of 20 or less.

z is an integer of 0 to 3, preferably an integer of 0 or more, and more preferably an integer of 1 or more. z is preferably an integer of 3 or less, and more preferably an integer of 1 or less.

* is an attachment position to any other constituent unit or an end group. As the end group, a hydrogen atom and a hydroxy group are exemplified, and a hydrogen atom is preferable.

The above-mentioned polymer represented by formula (2) and polymer represented by formula (3) may contain another constituent unit as long as the polymers does not deviate from the gist of the present invention. It is preferable that the above-mentioned polymer represented by formula (2) and polymer represented by formula (3) do not contain another constituent unit, or the rate of the other constituent unit be 3% by mass or less (preferably 1% by mass or less) of the polymer represented by formula (2) and the polymer represented by formula (3).

In the production method of the present embodiment, the polymer having the structure represented by formula (1) (moreover, the polymer represented by formula (2) or formula (3)) usually accounts for 90% by mass or more of polymer components that are raw materials, and preferably accounts for 95% by mass or more.

In the production method of the present embodiment, the polymer having the structure represented by formula (1) (moreover the polymer represented by formula (2) or formula (3)) may be used alone or in combination of two or more.

In the polymer having the structure represented by formula (1), the hydroxyl value, which is the mass of the polymer per 1 mol of hydroxy groups, is preferably 100 to 5,000 g/mol. The adjustment of the hydroxyl value to the above-mentioned lower limit value or more enables reducing the dielectric properties of the obtained (meth)acrylic acid ester compound. The adjustment of the hydroxyl value to the above-mentioned upper limit value or less enables introducing an enough amount of (meth)acrylic groups into the obtained (meth)acrylic acid ester compound to form a (meth)acrylic acid ester compound more excellent in heat resistance. The hydroxyl value is more preferably 2000 g/mol or less, and further preferably 1200 g/mol or less. The hydroxyl value is more preferably 200 g/mol or more, and further preferably 300 g/mol or more.

The hydroxyl value is measured according to the description of the Examples, described below.

The number average molecular weight of the polymer having the structure represented by formula (1) is preferably 1,000 to 10,000. If the number average molecular weight is in the range, the performance when the obtained (meth)acrylic acid ester compound is cured tends to be excellent in a balanced way. Specifically, the performance tends to be more excellent in low dielectric properties, heat resistance, ease of cure, uniform film formability, and the like. The number average molecular weight is more preferably 1,200 or more, and further preferably 1,500 or more. The number average molecular weight is more preferably 6,000 or less, further preferably 5,000 or less, still more preferably less than 4,000, and further still more preferably 3,500 or less.

The number average molecular weight (Mn) is measured according to the description of the Examples, described below.

The polymer having the structure represented by formula (1) to be used in the present embodiment preferably satisfies both the above-mentioned number average molecular weight and the hydroxyl value. In such a case, the effects of the present invention are more effectively exhibited.

The molecular weight distribution (Mw/Mn) is more preferably 1.01 or more, and further preferably 1.10 or more. The molecular weight distribution (Mw/Mn) is more preferably 10.0 or less, further preferably 5.00 or less, and still more preferably 3.00 or less. Especially in the present embodiment, even though the molecular weight distribution (Mw/Mn) is 1.50 or more, the hydroxy groups can be properly esterified.

The weight average molecular weight (Mw) is measured according to the description of the Examples, described below.

Then, the catalyst to be used in the production method of the present embodiment will be described. In the present embodiment, at least one of catalyst of potassium carbonate, rubidium carbonate, and cesium carbonate is used. The use of these catalysts enables promoting the reaction of the polymer having the structure represented by formula (1) with (meth)acrylic acid anhydride effectively. Furthermore, the obtained (meth)acrylic acid ester compound can be collected at a high yield.

In the present embodiment, among potassium carbonate, rubidium carbonate, and cesium carbonate, potassium carbonate and cesium carbonate are preferable, and potassium carbonate is more preferable.

Although the forms of potassium carbonate, rubidium carbonate, and cesium carbonate are not particularly prescribed, the forms are preferably powdered. The forms of potassium carbonate, rubidium carbonate, and cesium carbonate are preferably fine powders (average particle size: around 10 to 200 µm). The use of powdered potassium carbonate, rubidium carbonate, and cesium carbonate increases the specific surface area and enables enhancing the reactivity.

In the production method of the present embodiment, it is preferable to use 1.0 mol (mol/mol-OH) or more of at least one of potassium carbonate, rubidium carbonate, and cesium carbonate, it is more preferable to use 3.0 mol or more thereof, it is preferable to use 10.0 mol or less thereof, and it is more preferable to use 7.0 mol or less thereof in total per 1 mol of the hydroxy groups of the polymer having the structure represented by formula (1). When the amount of at least one of potassium carbonate, rubidium carbonate, and cesium carbonate is adjusted to the lower limit value or more, the reactivity of the hydroxy groups of the polymer having the structure represented by formula (1) with (meth)acrylic acid anhydride tends to be further improved. When the amount of at least one of potassium carbonate, rubidium carbonate, and cesium carbonate is adjusted to the upper limit value or less, the effect of reducing the production cost tends to be further improved.

In the production method of the present embodiment, only one of potassium carbonate, rubidium carbonate, and cesium carbonate may be used, or two or more thereof may be used. If the two or more are used, the total amount is preferably in the above-mentioned range.

In the production method of the present embodiment, the esterification reaction can be performed without using 4-dimethylaminopyridine (DMAP), which has been commonly used until now. That is, in the production method of the present embodiment, the esterification reaction can be advanced under the condition that DMAP is substantially absent. Under the condition that DMAP is substantially absent, the abundance of DMAP is preferably 0.1 mol or less, more preferably 0.05 mol or less, further preferably 0.03 mol or less, and still more preferably 0.01 mol or less with respect to 1.0 mol of potassium carbonate, rubidium carbonate, and cesium carbonate in total. The lower limit value is 0 mol.

In the production method of the present embodiment, the esterification reaction is preferably advanced under the condition that an esterification catalyst other than potassium carbonate, rubidium carbonate, and cesium carbonate is substantially absent. Under the condition that the esterification catalyst is substantially absent, the abundance of the esterification catalyst other than potassium carbonate, rubidium carbonate, and cesium carbonate is preferably 0.1 mol or less, more preferably 0.05 mol or less, further preferably 0.03 mol or less, and still more preferably 0.01 mol or less with respect to 1.0 mol of potassium carbonate, rubidium carbonate, and cesium carbonate in total. The lower limit value is 0 mol. The adjustment of the abundance to such a range enables isolating the (meth)acrylic acid ester compound at a higher yield.

In the present embodiment, the polymer having the structure represented by formula (1) is reacted with (meth)acrylic acid anhydride. (Meth)acrylic acid anhydride is methacrylic acid anhydride and/or acrylic acid anhydride, and methacrylic acid anhydride is preferable. The use of methacrylic acid anhydride exhibits the effect of improving the heat resistance more effectively when a thermosetting resin is produced.

In the production method of the present embodiment, the ratio of X substituted with (meth)acrylic groups to the X of the polymer having the structure represented by formula (1) (rate of (meth)acrylic groups introduced into the phenolic hydroxy groups) is preferably 85% by mol or more, more preferably 90% by mol or more, further preferably 93% by mol or more, and still more preferably 97% by mol or more. The upper limit is ideally 100% by mol, but practically 99.9% by mol or less.

In the esterification reaction, it is preferable to use 1.0 mol or more of (meth)acrylic acid anhydride, and it is more preferable to use 1.1 mol or more thereof per 1 mol of the hydroxy groups of the polymer having the structure represented by formula (1). When the amount of (meth)acrylic acid anhydride is adjusted to the lower limit value or more, the hydroxy groups of the polymer having the structure represented by formula (1) tend to be converted into (meth)acrylic groups at a higher rate. The amount of (meth)acrylic acid anhydride is preferably 10.0 mol or less, more preferably 8.0 mol or less, further preferably 5.0 mol or less, still more preferably 4.5 mol or less, further still more preferably 3.0 mol or less, and further much more preferably 2.0 mol or less per 1 mol of the hydroxy groups of the polymer having the structure represented by formula (1). The adjustment of the amount to the upper limit value or less facilitates the purification and enables reducing the production cost more effectively.

If both methacrylic acid anhydride and acrylic acid anhydride are used in the production method of the present embodiment, the total amount thereof is in the above-mentioned range.

In the production method of the present embodiment, the polymer having the structure represented by formula (1) is further reacted with an acyl compound, and the polymer may have a configuration in which some of X after the reaction are acyl groups. If the polymer having the structure represented by formula (1) to be used in the production method of the present embodiment contains many phenolic hydroxy groups, the reaction with only (meth)acrylic acid anhydride introduces many (meth)acrylic groups. However, if the (meth)acrylic acid ester compound obtained in the production method of the present embodiment is used as a low dielectric material, the introduction of (meth)acrylic groups more than required into the (meth)acrylic acid ester compound may increase the dielectric constant and the dielectric loss tangent. It may be difficult to select only a raw material oligomer (polymer having the structure represented by formula (1)) containing a small amount of the phenolic hydroxy groups. In such a case, the polymer having the structure represented by formula (1) is also reacted with the acyl compound in addition to (meth)acrylic acid anhydride, and a configuration in which some of X after the reaction are (meth)acrylic groups, and the others are acyl groups is formed to obtain a desired low dielectric material.

If the acyl compound is reacted in addition to (meth)acrylic acid anhydride in the production method of the present embodiment, (meth)acrylic acid anhydride and the acyl compound may be reacted at the same time, or either thereof may be reacted before the other is reacted. In the production method of the present embodiment, it is preferable to react (meth)acrylic acid anhydride and then react the acyl compound. Reacting (meth)acrylic acid anhydride and then reacting the acyl compound enable obtaining a polymer having a low residual hydroxy group rate while having (meth)acrylic groups in an amount suitable for the production of a low dielectric material. Although the acylation reaction using the acyl compound is also preferably performed in the same reaction system, the acylation reaction may be performed in a reaction system in which potassium carbonate, rubidium carbonate, and cesium carbonate is substantially absent. For example, the polymer having a structure represented by formula (1) is reacted with (meth)acrylic acid anhydride, the reaction product is then collected, the acyl compound may be reacted in a different reaction system.

Although the type and the like of the acyl compound are not particularly prescribed, acetic acid anhydride is preferable from the viewpoint of production cost. Although acetic acid anhydride was reacted in the present Example 9 to substitute hydroxy groups with methacrylic groups and acetyl groups at a molar ratio of 1:1, hydroxy groups can be substituted at any ratio in view of the amount of the hydroxyl equivalent of the raw material resin to be reacted.

If the acyl compound is reacted in the production method of the present embodiment, the ratio of X substituted with acyl groups to the X of the polymer having the structure represented by formula (1) is preferably 5% by mol or more, and more preferably 10% by mol or more. When the ratio is adjusted to the lower limit value or more, a resin having better dielectric properties tends to be able to be obtained. The ratio of X substituted with acyl groups to the X of the polymer having the structure represented by formula (1) is preferably 90% by mol or less, and more preferably 80% by mol or less. When the ratio is adjusted to the upper limit value or less, the thermosetting properties of the polymer having the structure represented by formula (1) can be maintained more satisfactorily.

In the esterification reaction, it is preferable to use 0.1 mol or more of the acyl compound, and it is more preferable to use 0.2 mol or more thereof per 1 mol of hydroxy groups of the polymer having the structure represented by formula (1). The adjustment of the amount of the acyl compound to the lower limit value or more enables reducing the hydroxy group residual rate of the polymer having the structure represented by formula (1) effectively. It is preferable that the amount of the acyl compound be 10 mol or less, and it is more preferable that the amount be 5 mol or less per 1 mol of hydroxy groups of the polymer having the structure represented by formula (1). When the amount of the acyl compound is adjusted to the upper limit value or less, the purification is more easily performed, and the production cost tends to be reduced.

The acyl compound may be used alone or in combination of two or more. If two or more thereof are used, the total amount is preferably in the above-mentioned range.

In the production method of the present embodiment, the esterification reaction is preferably performed in the presence of a solvent. If the polymer having the structure represented by formula (1) is liquid in the production method of the present embodiment, a solvent does not need to be used. However, if the polymer having the structure represented by formula (1) is non-liquid, a solvent is usually used. The use of the solvent enables advancing the ionization of potassium carbonate, rubidium carbonate, and cesium carbonate and the ionization of the phenolic hydroxy groups of the polymer having the structure represented by formula (1) effectively.

As long as the solvent to be used in the production method of the present embodiment dissolves the polymer having the structure represented by formula (1), and does not inhibit the esterification reaction in the present embodiment markedly, the solvent can be used without particular limitation. However, an aprotic solvent is preferable, and at least one of an aromatic hydrocarbon solvent and an ether solvent is more preferable. Action on O⁻ derived from phenolic hydroxy groups tends to proceed effectively using the aprotic solvent.

As the solvent, toluene, dimethylacetamide (DMAC), cyclopentyl methyl ether (CPME), 4-methyltetrahydropyran (MTHP), and 1,4-dioxane (dioxane) are specifically exemplified. Toluene is preferable as the solvent from the viewpoint that toluene is low polar, is a good solvent of the polymer having a structure represented by formula (1), and has a boiling point suitable for the esterification reaction. Cyclopentyl methyl ether (CPME), which is highly hydrophobic and hardly produce superoxides, is preferable from the viewpoint that the solvent is efficiently collected and reused.

As the solvent, a dehydration solvent is preferable. The use of the dehydration solvent enables increasing the yield of the obtained (meth)acrylic acid ester compound.

If the solvent is used in the production method of the present embodiment, the amount thereof is preferably 0.1 mL or more, more preferably 1.0 mL or more, and further preferably 2.0 mL or more with respect to 1 g of the polymer having a structure represented by formula (1). The adjustment of the amount to the lower limit value or more enables effectively securing the fluidity for advancing the esterification reaction smoothly. The amount of the solvent is preferably 200 mL or less, more preferably 100 mL or less, and further preferably 50 mL or less with respect to 1 g of the polymer having the structure represented by formula (1). When the amount is adjusted to the upper limit value or less, the concentration for advancing the esterification reaction smoothly can be maintained, and the effect of reducing the production cost tends to be further improved.

In the production method of the present embodiment, the solvent may be used alone or in combination of two or more. If two or more thereof are used, the total amount thereof is preferably in the above-mentioned range.

Although the (meth)acrylic acid ester compound obtained in the present embodiment may be a methacrylic acid ester compound or an acrylic acid ester compound, the (meth)acrylic acid ester compound is preferably a methacrylic acid ester compound.

In the present embodiment, the reaction temperature of the esterification reaction is preferably -20°C or more, more preferably 0°C or more, and further preferably 20°C or more. When the reaction temperature is adjusted to the lower limit value or more, the esterification reaction tends to be advanced smoothly to convert the hydroxy groups of the polymer having the structure represented by formula (1) into (meth)acrylic groups at a high rate. The reaction temperature of the esterification reaction is preferably 200°C or less, more preferably 180°C or less, and further preferably 150°C or less. When the reaction temperature is adjusted to the upper limit value or less, (meth)acrylic groups tend to be prevented from causing side reactions such as polymerization, and the safety of the production tends to be able to be enhanced.

In the present embodiment, the reaction time of the esterification reaction is preferably 0.5 hours or more, more preferably 1.0 hour or more, and further preferably 2.0 hours or more. When the reaction time is adjusted to the lower limit value or more, the hydroxy groups of the polymer having the structure represented by formula (1) tend to be converted into (meth)acrylic groups at a high rate. The reaction time of the esterification reaction is preferably 120 hours or less, more preferably 72 hours or less, and further preferably 48 hours or less. When the reaction time is adjusted to the upper limit value or less, the effect of reducing the production cost tends to be further improved.

In the present embodiment, the esterification may be performed in the usual atmosphere (in the presence of air), or may be performed in an inert gas atmosphere. The production cost can be further reduced by performing the esterification in the usual atmosphere. The esterification in an inert atmosphere enables advancing the esterification reaction in a nonaqueous system or a deoxidized system and enables advancing the esterification more effectively.

In the production method of the present embodiment, filtration is preferably performed after the esterification reaction. In the production method of the present embodiment, the (meth)acrylic acid ester compound can be collected in a high recovery by one filtration. The diameter of the filter for filtration in the present embodiment is preferably 0.2 to 7.0 µm. It is preferable that the reaction system be cooled to around room temperature (for example, 20 to 40°C), and the filtration be performed after the esterification reaction. It is preferable that the reaction liquid after the filtration be vacuum-dried, and it is more preferable that most of the reaction solvent be distilled off, then the reaction liquid be vacuum-dried.

As described above, it is needless to say that, in the production method of the present embodiment, a (meth)acrylic acid ester compound can be isolated at a high recovery only by filtration operation, but liquid separation operation and recrystallization operation, or other purification operations may be performed for further increasing the purity.

In the production method of the present embodiment, the amount of the (meth)acrylic acid that is an impurity is preferably less than 1% by mol, more preferably 0.8% by mol or less, further preferably 0.6% by mol or less, and still more preferably 0.4% by mol or less with respect to 1 mol of the polymer having the structure represented by formula (1). The lower limit value of the amount of (meth)acrylic acid that is the impurity is ideally 0% by mol, but practically 0.01% by mol or more.

If the polymer having the structure represented by formula (1) is also reacted with the acyl compound in addition to (meth)acrylic acid anhydride in the production method of the present embodiment, the amount of an impurity derived from the acyl compound is preferably less than 1% by mol, more preferably 0.8% by mol or less, further preferably 0.6% by mol or less, and still more preferably 0.4% by mol or less with respect to 1 mol of the polymer having the structure represented by formula (1). The lower limit value of the amount of an impurity derived from the acyl compound that is the impurity is ideally 0% by mol, but practically 0.01% by mol or more. For example, if the acyl compound is acetic acid anhydride, the impurity derived from the acyl compound is acetic acid.

### <Uses>

The (meth)acrylic acid ester compound obtained in the production method of the present embodiment may be used as it is, or may be used as a resin composition in which other curable compounds and additives are blended therewith. As the other curable compounds, compounds having carbon-carbon unsaturated bond groups and epoxy resins are exemplified. Examples of the additives include flame retardants, ultraviolet absorbents, antioxidants, photopolymerization initiators, fluorescent whitening agents, photosensitizers, dyes, pigments, thickener, flow regulator, lubricants, antifoaming agents, dispersants, leveling agents, brighteners, and polymerization inhibitors.

The (meth)acrylic acid ester compound obtained by the production method of the present embodiment or a resin composition containing the (meth)acrylic acid ester compound is preferably cured and used as a cured product. Since such a cured product is excellent in heat resistance and dielectric properties, the cured product can be suitably used as the insulating layer of a printed-wiring board or a material for a semiconductor package.

### [Examples]

Hereinafter, the present invention will be described further specifically by giving the Examples. As long as materials, the used amounts, the rates, the contents of treatments, the procedures of treatments, and the like shown in the following Examples do not deviate from the gist of the present invention, the materials, the used amounts, the rates, the contents of treatments, the procedures of treatments, and the like can be appropriately changed. Accordingly, the scope of the present invention is not limited to specific examples shown below.

If a measuring apparatus and the like used in the Examples are difficultly obtained due to the discontinuance or the like, measurement can be performed using another apparatus having equivalent performance.

The number average molecular weight (Mn) and the molecular weight distribution (Mw/Mn) of a raw material resin were calculated in terms of standard polystyrene using gel permeation chromatography (GPC) . The used devices were an integrated LC (LC-2010 _{HT} manufactured by SHIMADZU CORPORATION) and an RI detector (RID-20A manufactured by SHIMADZU CORPORATION), and the column was a column in which a guard column (KF-G 4A) manufactured by Resonac America, Inc. and four standard columns (KF-801, KF-802, KF-803, and KF-804) for GPC manufactured by Resonac America, Inc. were connected in series. A sample dissolved in THF (tetrahydrofuran) was passed therethrough and measured under the condition that the eluent was THF, the flow rate was 1.0 mL, and the temperature was 40°C.

### Example 1

First, 828 mg of oligo phenylene ether having hydroxy groups at both ends (produced by SABIC, SA-90, in the group R^{a}, na + ma is around 12.) (amount of hydroxy groups: 1 mmol) was reacted with 185 mg (1.2 mmol) of methacrylic acid anhydride (MAA) in the presence of 691 mg (5 mmol) of potassium carbonate (produced by FUJIFILM Wako Pure Chemical Corporation, average particle size: 150 µm or less) at 100°C for 6 hours using toluene (8 mL) as a solvent according to the following scheme.

Then, 0.5 mL of the reaction liquid was air-cooled, then filtered through a 0.45-µm filter, and vacuum-dried (pressure: less than 1 hPa, 40°C) for 1 hour. This was dissolved in deuterated chloroform, and the methacrylation rate (OH modification rate) and the methacrylic acid residual rate (impurities) were calculated by proton nuclear magnetic resonance spectrum (¹H NMR) analysis according to the knack described below. It was confirmed that methacrylic groups were introduced into the hydroxy groups which the oligo phenylene ether having hydroxy groups in both ends had at a high conversion rate of 94% by mol of methacrylic groups.

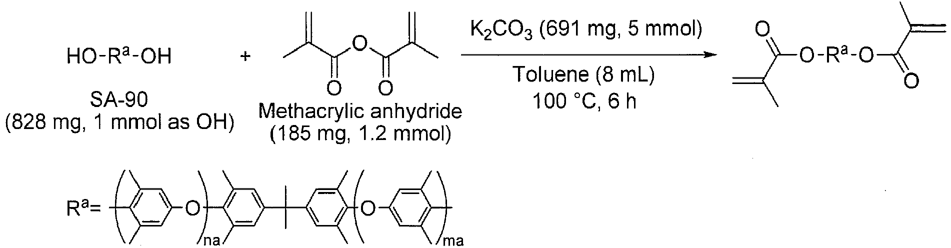

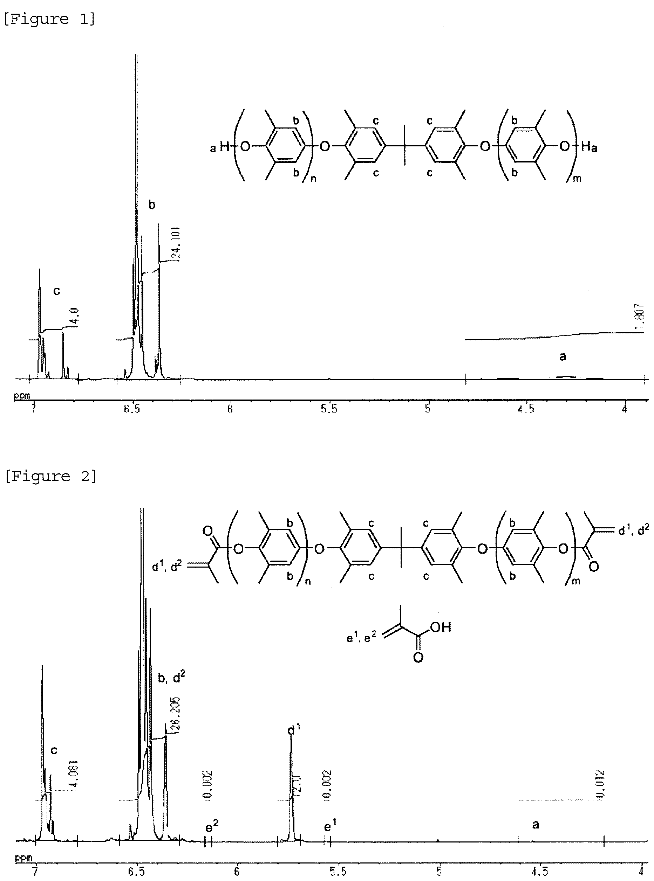

The methacrylation rate (OH modification rate) and the methacrylic acid residual rate (rate of impurities) of the polymer were calculated based on proton nuclear magnetic resonance spectrum (¹H NMR) analysis results. As the nuclear magnetic resonance device, an AVANCE III 500 (500 MHz), manufactured by BRUKER, was used. Specifically, 0.5 mL of the reaction liquid was air-cooled and then filtered through a 0.45-µm filter, and the filtrate was vacuum-dried (pressure: less than 1 hPa, 40°C) for 1 hour. This was dissolved in deuterated chloroform, and the solution was subjected to ¹H NMR analysis at 25°C. Here, Figure 1 shows a ¹H NMR spectrum (in the range of 4 ppm to 7 ppm) of SA-90, which is an oligo phenylene ether having hydroxy groups on both ends. In Figure 1, although the theoretical value of the integral ratio of the protons of the hydroxy groups at around 4.1 to 4.6 ppm (a) should be 2.0 with respect to the integral ratio of the protons bound to the aromatic rings of a core at around 6.8 to 7.0 ppm (c) (4.0), it was actually observed that the integral ratio was 1.8. This is partly because the protons of the hydroxy groups are easily subjected to proton exchange in the solution, and the peak becomes broad, and is difficultly observed. Hydroxy groups are easily subjected to intermolecular hydrogen bonding and the like, and the quantification is often difficult also by an analytical method other than ¹H NMR. Then, the methacrylation rate and the methacrylic acid residual rate were calculated in the comparison with the integral ratio of the protons bound to the aromatic rings of repeating structural units of the polymer (b) (24.1).

The hydroxyl value of the raw material resin was also determined by NMR analysis (integral ratios of the protons (b) and the protons (c)) according to the above-mentioned method. The calculated hydroxyl value was a value equivalent to the standard value of the product of the raw material resin.

### Example 2

In Example 1, the catalyst was changed from 5 mmol of potassium carbonate to 5 mmol of cesium carbonate (1.63 g), and the others were performed in the same way. It was confirmed that methacrylic groups were introduced into the hydroxy groups that the oligo phenylene ether having hydroxy groups at both ends had at a high conversion rate of 96% by mol. The methacrylation rate and the methacrylic acid residual rate were measured according to Example 1.

### Example 3

In Example 1, the solvent was changed into dimethylacetamide (DMAC), and the others were performed in the same way. It was confirmed that methacrylic groups were introduced into the hydroxy groups that the oligo phenylene ether having hydroxy groups at both ends had at a high conversion rate of 90% by mol. The methacrylation rate and the methacrylic acid residual rate were measured according to Example 1.

### Example 4

In Example 1, the solvent was changed into 1,4-dioxane (dioxane), and the others were performed in the same way. It was confirmed that methacrylic groups were introduced into the hydroxy groups that the oligo phenylene ether having hydroxy groups at both ends had at a high conversion rate of 99% by mol. The methacrylation rate and the methacrylic acid residual rate were measured according to Example 1.

### Example 5

In Example 1, the solvent was changed into cyclopentyl methyl ether (CPME), and the others were performed in the same way. It was confirmed that methacrylic groups were introduced into the hydroxy groups that the oligo phenylene ether having hydroxy groups at both ends had at a high conversion rate of more than 99% by mol.

Figure 2 shows a ¹H NMR spectrum (in the range of 4 ppm to 7 ppm) of the obtained oligo phenylene ether having methacrylic groups at both ends. When the integral ratios of the protons of the double bonds of the methacrylic groups near 5.7 to 5.8 ppm and near 6.3 to 6.4 ppm (d¹, d²) in Figure 2 were each defined as 2.0, the methacrylation rate was calculated as follows: 24.1/(26.2 - 2.0) × 100 = more than 99%. When the hydroxy group residual rate was calculated from the integral ratio of the protons of hydroxy groups near 4.1 to 4.6 ppm (a) (0.012), 0.012/(2.0 + 0.012) × 100 = 0.6%, and the result was almost consistent with the methacrylation rate (more than 99%) . The methacrylic acid residual rate per 1 molecule of the polymer was calculated from the integral ratios of the protons of the double bonds of methacrylic acid near 5.5 to 5.6 ppm and near 6.1 to 6.2 ppm (e¹, e²) (0.002, 0.002) as follows: 0.002 × 24.1/(26.2 - 2.0) × 100 = 0.2%.

### Example 6

In Example 1, the solvent was changed into 4-methyltetrahydropyran (MTHP), and the others were performed in the same way. It was confirmed that methacrylic groups were introduced into the hydroxy groups that the oligo phenylene ether having hydroxy groups at both ends had at a high conversion rate of 99% by mol. The methacrylation rate and the methacrylic acid residual rate were measured according to Example 1.

### Example 7

In Example 1, the oligo phenylene ether (produced by SABIC, SA-90) having hydroxy groups at both ends was changed into 821 mg of OPE-2000 (produced by MITSUBISHI GAS CHEMICAL COMPANY, INC., in the group R^{b}, nb + mb is around 12.) (amount of hydroxy groups: 1 mmol), and the others were performed in the same way. It was confirmed that methacrylic groups were introduced into the hydroxy groups of the oligo phenylene ether at a high conversion rate of 95% by mol. The methacrylation rate and the methacrylic acid residual rate were measured according to Example 1.

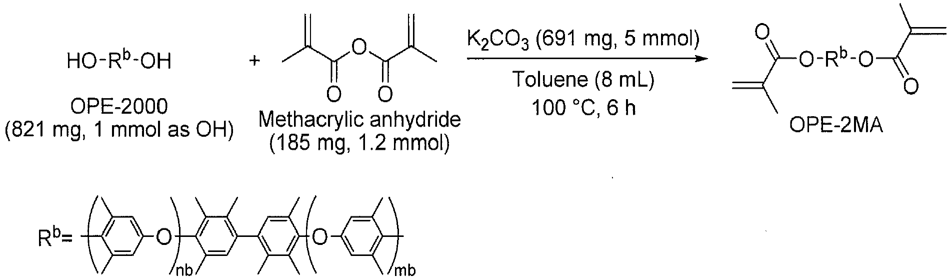

### Example 8

In Example 7, the solvent was changed into cyclopentyl methyl ether (CPME), and the others were performed in the same way. It was confirmed that methacrylic groups were introduced into the hydroxy groups of the oligo phenylene ether at a high conversion rate of 98% by mol. The methacrylation rate and the methacrylic acid residual rate were measured according to Example 1.

### Example 9

According to the following scheme, 3.46 g of p-tert-butyl-modified xylene resin (Xy-PTBP) (produced by MITSUBISHI GAS CHEMICAL COMPANY, INC., nc is around 6, and mc is around 4.) (amount of hydroxy groups: 10 mmol) was reacted with 6 mmol of methacrylic acid anhydride (MAA) (925 mg) in the presence of 50 mmol of potassium carbonate (6.91 g) at 100°C for 6 hours using cyclopentyl methyl ether (CPME) as the solvent (8 mL). The resultant was further reacted with 32 mmol of acetic acid anhydride (Ac₂O) (3.27 g) at 100°C for 6 hours.

Then, 0.5 mL of the reaction liquid was air-cooled, then filtered through a 0.45-µm filter, and vacuum-dried (pressure: less than 1 hPa, 40°C) for 1 hour. This was dissolved in deuterated chloroform, and the methacrylation rate and the methacrylic acid residual rate were calculated by proton nuclear magnetic resonance spectrum (¹H NMR) analysis. Methacrylic groups and acetyl groups were introduced into the hydroxy groups that Xy-PTBP had at more than 99% by mol in total so that the ratio of methacrylic groups to acetyl groups was 1:1 (molar ratio). nd in Xy-PTBP-MA-Ac was around 6, md was around 2, and ld was around 2. The methacrylation rate and the methacrylic acid residual rate were measured according to Example 1.

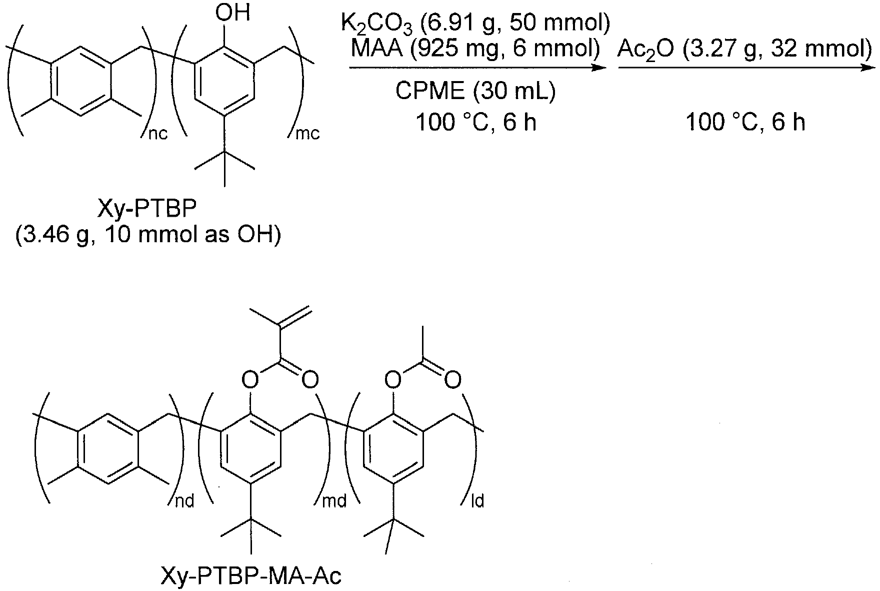

### Comparative Example 1

In Example 1, potassium carbonate was changed into 0.2 mmol of 4-dimethylaminopyridine (DMAP), and the others were performed in the same way. The rate of the methacrylic groups introduced into the hydroxy groups that the hydroxy group-containing oligo phenylene ether had was 92% by mol.

Unlike the cases of Examples, even though the resultant was filtered after the reaction, most of the DMAP (37% with respect to 1 molecule of the polymer) and the by-produced methacrylic acid (137% with respect to 1 molecule of polymer) remained.

Since DMAP was expensive, the method was more disadvantageous than the potassium carbonate method in view of cost.

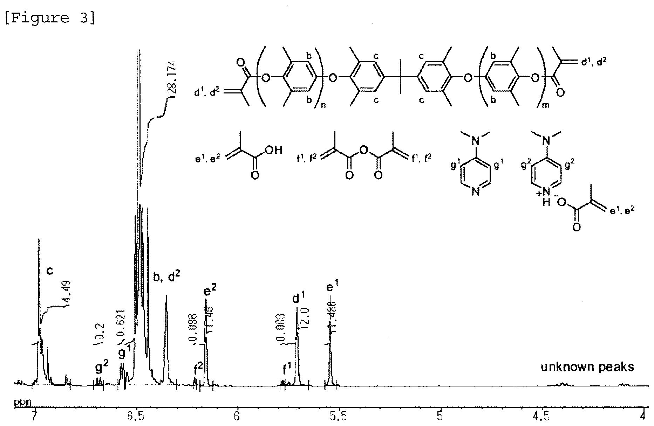

Figure 3 shows a ¹H NMR spectrum (in the range of 4 ppm to 7 ppm) of the oligo phenylene ether having methacrylic groups at both ends obtained in Comparative Example 1. When the integral ratios of the protons of the double bonds of the methacrylic groups near 5.7 to 5.8 ppm and near 6.3 to 6.4 ppm (d¹, d²) in Figure 3 were each defined as 2.0, the methacrylation rate was calculated as follows: 24.1/(28.2 - 2.0) × 100 = 92%. The protons of the hydroxy groups near 4.1 to 4.6 ppm (a) were not observed due to proton exchange with a large amount of methacrylic acid existing in the solution. Some peaks of unknown structures were observed instead. The methacrylic acid residual rate per 1 molecule of the polymer was calculated from the integral ratios of the protons of the double bonds of methacrylic acid near 5.5 to 5.6 ppm and near 6.1 to 6.2 ppm (e¹, e²) (1.49, 1.49) as follows: 1.49 × 24.1/(28.2 - 2.0) × 100 = 137%. The DMAP residual rate per 1 molecule of the polymer was calculated from the integral ratio of the protons bound to the heterocycle of DMAP near 6.6 ppm (g¹) (0.6) as follows: (0.6/2) × 24.1/(28.2 - 2.0) × 100 = 28%. The residual rate of DMAP to which methacrylic acid was added per 1 molecule of the polymer was calculated from the integral ratio of the protons bound to the heterocycle of DMAP-methacrylate near 6.7 ppm (g²) (0.2) as follows: (0.2/2) × 24.1/(28.2 - 2.0) × 100 = 9%. The residual rate of methacrylic acid anhydride per 1 molecule of the polymer was calculated from the integral ratios of the protons of the double bonds of methacrylic acid anhydride near 5.8 ppm and near 6.2 ppm (f¹, f²) (0.09, 0.09) as follows: (0.09/2) × 24.1/(28.2 - 2.0) × 100 = 4%.

### Comparative Example 2

In Example 1, potassium carbonate was changed into 5 mmol of sodium carbonate, and the others were performed in the same way. The rate of methacrylic groups introduced into the hydroxy groups that the hydroxy group-containing oligo phenylene ether had was 21% by mol. The methacrylation rate and the methacrylic acid residual rate were measured according to Comparative Example 1.

### Comparative Example 3

In Example 1, potassium carbonate was changed into 5 mmol of lithium carbonate, and the others were performed in the same way. The rate of methacrylic groups introduced into the hydroxy groups that the hydroxy group-containing oligo phenylene ether had was 5% by mol. The methacrylation rate and the methacrylic acid residual rate were measured according to Comparative Example 1.

### Comparative Example 4

In Example 1, potassium carbonate was changed into 5 mmol of calcium carbonate, and the others were performed in the same way. The rate of methacrylic groups introduced into the hydroxy groups that the hydroxy group-containing oligo phenylene ether had was 3% by mol. The methacrylation rate and the methacrylic acid residual rate were measured according to Comparative Example 1.

### Comparative Example 5

In Example 1, potassium carbonate was changed into 5 mmol of potassium hydrogen carbonate, and the others were performed in the same way. The rate of methacrylic groups introduced into the hydroxy groups that the hydroxy group-containing oligo phenylene ether had was 7% by mol. The methacrylation rate and the methacrylic acid residual rate were measured according to Comparative Example 1.

### Comparative Example 6

In Example 1, potassium carbonate was changed into 5 mmol of potassium hydroxide, and the others were performed in the same way. The introduction of methacrylic groups into the hydroxy groups that the hydroxy group-containing oligo phenylene ether had was not observed. The methacrylation rate and the methacrylic acid residual rate were measured according to Comparative Example 1.

A correlation was seen between the ionic radius of the alkali metal carbonate and the reactivity of methacrylation from Examples and Comparative Examples 2 and 3, and it was observed that the use of sodium carbonate, having a smaller ionic radius than potassium carbonate, greatly reduced the conversion rate. It was conjectured that as the ionic radius of the metal cation increased, the nucleophilic attacking properties on MAA increased due to the naked state of phenoxy anions at the time of activating phenolic hydroxy groups.

Also, in alkaline earth metal carbonates in the same way, the use of calcium carbonate, having an ionic radius equivalent to that of lithium carbonate, allows the methacrylation conversion rate to be a very low conversion rate of 3% by mol (Comparative Example 4). Even though potassium hydrogen carbonate, which was a bicarbonate, was used, the methacrylation conversion rate was a very low conversion rate of 5% by mol (Comparative Example 5). Although potassium hydroxide is strongly basic, MAA was decomposed under the influence of the hydroxide ions thereof, and the reaction did not proceed at all (Comparative Example 6).

### Comparative Example 7

In Example 1, methacrylic acid anhydride (MAA) was changed into 1.2 mmol of methacrylic acid chloride (MAC) (125 mg), and the others were performed in the same way. The rate of methacrylic groups introduced into the hydroxy groups that the oligo phenylene ether having hydroxy groups at both ends had was 46% by mol. Although methacrylic acid chloride (MAC) has higher reactivity than MAA, the decomposability due to a minute amount of water and the like in the reaction system was also high, all MAC was therefore consumed at a conversion rate of around 50 percent. The methacrylation rate and the methacrylic acid residual rate were measured according to Comparative Example 1.

### Comparative Example 8

According to the following scheme, 2.07 g of an oligo phenylene ether having hydroxy groups at both ends (produced by SABIC, SA-90) (amount of hydroxy groups: 2.5 mmol) was reacted with 5 mmol of methacrylic acid (MA) using para-xylene (20 mL) as a solvent in the presence of 0.5 mmol of B(OH)₃/H₂SO₄ with a reactor to which a Dean-Stark apparatus was attached at 130°C for 24 hours while the reaction liquid was azeotropically distilled.

Then, 0.5 mL of the reaction liquid was air-cooled, then filtered through a 0.45-µm filter, and vacuum-dried (pressure: less than 1 hPa, 40°C) for 1 hour. This was dissolved in deuterated chloroform, and the methacrylation rate and the methacrylic acid residual rate were calculated by proton nuclear magnetic resonance spectrum (¹H NMR) analysis. The rate of methacrylic groups introduced into the hydroxy groups that the oligo phenylene ether having hydroxy groups at both ends had was 4% by mol. The methacrylation rate and the methacrylic acid residual rate were measured according to Comparative Example 1.

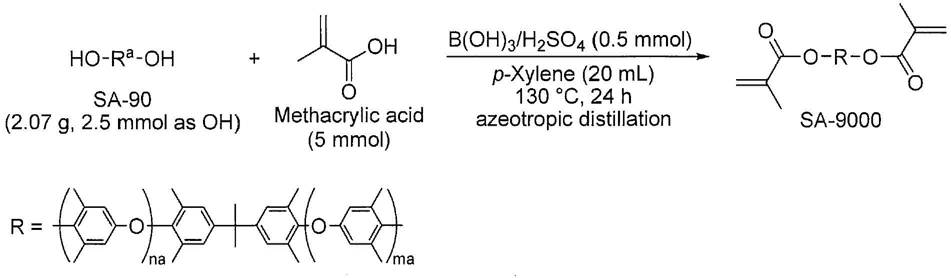

### Comparative Example 9

In Comparative Example 8, 0.25 mmol of p-Me-C₆H₄SO₃H•H₂O (para-toluenesulfonic acid monohydrate, PTSA-H₂O) was used instead of B(OH)₃/H₂SO₄, the amount of methacrylic acid (MA) was changed from 5 mmol to 12.5 mmol, and the others were performed in the same way. The rate of methacrylic groups introduced into the hydroxy groups that the oligo phenylene ether having hydroxy groups at both ends had was 6% by mol. The methacrylation rate and the methacrylic acid residual rate were measured according to Comparative Example 1.

### Comparative Example 10

In Comparative Example 8, AMBERLYST(R) 15 (produced by Merck KGaA) was used instead of B(OH)₃/H₂SO₄ in an amount of 10% by mass with respect to the mass of the oligo phenylene ether having hydroxy groups at both ends (produced by SABIC, SA-90), the amount of methacrylic acid (MA) was changed from 5 mmol to 12.5 mmol, and the others were performed in the same way. The rate of methacrylic groups introduced into the hydroxy groups that the oligo phenylene ether having hydroxy groups at both ends had was 6% by mol.

In Comparative Example 8, SA-90 was methacryl-modified with MA in a B(OH)₃/H₂SO₄ cocatalyst system. Consequently, when the reaction temperature was adjusted to 130°C in a xylene solvent, the progress of the reaction was observed. However, even though MA and B(OH)₃/H₂SO₄ were used in 1.7 times and 3 or more times the amounts in Example described in Japanese Patent Laid-Open No. 2011-105667, respectively, the conversion rate was a very low rate of 4%. As causes for the methacrylation reaction to be low efficient as compared with Example described in Japanese Patent Laid-Open No. 2011-105667, a decrease in reactivity due to the steric hindrance of 2,6-dimethyl groups adjoining the phenolic hydroxy groups at the ends of SA-90 and the inhibition of the catalytic activity by the interaction between B(OH)₃/H₂SO₄ and the oligo phenylene ether group of the SA-90 main chain were conjectured. Additionally, the use of para-toluenesulfonic acid and AMBERLYST15, namely a solid acid, commonly used as acid catalysts for esterification, allows the conversion rate to be a low conversion rate of around 6% even using a large amount of MA (12.5 mmol) (Comparative Examples 9 and 10).

### Example 10

As shown in the following scheme, the reaction was performed in the same way on a 25 times scale in Example 8. It was confirmed that methacrylic groups were introduced into the hydroxy groups of the oligo phenylene ether at a high conversion rate of 95% by mol. The obtained compound was beige and powdered, and the residual hydroxy group amount was 5% by mol.

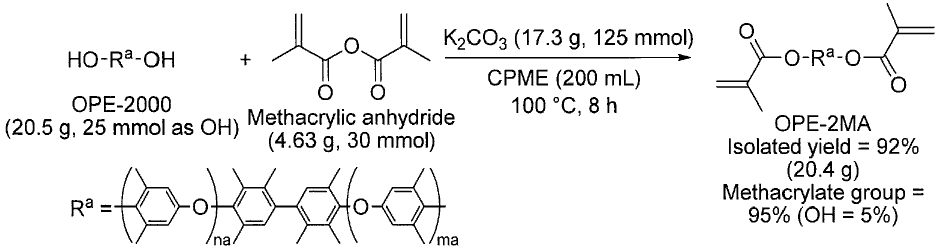

### Comparative Examples 11 to 13

In Example 1, the type of the catalyst was changed into sodium acetate (AcONa), and the amount of the catalyst and the type of the solvent were changed as shown in Table 5, and the others were performed in the same way. Table 5 shows the results.

The results of Examples 1 to 9 and Comparative Examples 1 to 13 are summarized as follows. In the following tables, the impurities indicate methacrylic acid (MA) unless otherwise specified. The AcOH indicates acetic acid. The amount of impurities may be larger than 100% by mol because, for example, in the case where 1.2 mmol of methacrylic acid anhydride is used with respect to 1 mmol of the hydroxy groups, 240% by mol methacrylic acid anhydride is added to 1 molecule of the polymer. Since the values of the residual hydroxy groups and the introduced methacrylic groups vary from one of Examples and Comparative Examples to another thereof, the attempt to calculate the amount of the impurities with respect to the hydroxy groups and the methacrylic groups makes the comparison difficult. Therefore, the amount of the impurities is calculated as a value with respect to 1 molecule of the polymer.

**[Table 1]**

| | | | Example 1 | Example 2 | Example 3 | Example 4 | Example 5 |
|---|---|---|---|---|---|---|---|
| Raw material resin | | | SA90 | SA90 | SA90 | SA90 | SA90 |
| | Molecular weight | (Mn) | 2242 | 2242 | 2242 | 2242 | 2242 |
| | Molecular weight distribution | (Mw/Mn) | 1.68 | 1.68 | 1.68 | 1.68 | 1.68 |
| | Hydroxyl value | (g/mol) | 828 | 828 | 828 | 828 | 828 |
| Reaction solvent | | | Toluene | Toluene | DMAC | Dioxane | CPME |
| (Meth)acrylic acid anhydride, acyl compound, and compound for comparison | | | MAA | MAA | MAA | MAA | MAA |
| | Added amount | (mol/mol-OH) | 1.2 | 1.2 | 1.2 | 1.2 | 1.2 |
| Catalyst | | | K2CO3 | Cs2CO3 | K2CO3 | K2CO3 | K2CO3 |
| | Added amount | (mol/mol-OH) | 5.0 | 5.0 | 5.0 | 5.0 | 5.0 |
| | Reaction temperature | (°C) | 100 | 100 | 100 | 100 | 100 |
| | Reaction time | (Hour) | 6.0 | 6.0 | 6.0 | 6.0 | 6.0 |
| OH modification rate (introduction rate) | | (% by mol) | 94 | 96 | 90 | 99 | >99 |
| | Impurity | (% by mol) | 0.2 | 0.1 | 0.3 | 0.3 | 0.2 |

**[Table 2]**

| | | | Example 6 | Example 7 | Example 8 | Example 9 |
|---|---|---|---|---|---|---|
| Raw material resin | | | SA90 | OPE2000 | OPE2000 | Xy-PTBP |
| | Molecular weight | (Mn) | 2242 | 2250 | 2250 | 1295 |
| | Molecular weight distribution | (Mw/Mn) | 1.68 | 1.72 | 1.72 | 1.82 |
| | Hydroxyl value | (g/mol) | 828 | 821 | 821 | 346 |
| Reaction solvent | | | MTHP | Toluene | CPME | CPME |
| (Meth)acrylic acid anhydride, acyl compound, and compound for comparison | | | MAA | MAA | MAA | MAA, Ac₂O |
| | Added amount | (mol/mol-OH) | 1.2 | 1.2 | 1.2 | 0.6, 3.2 |
| Catalyst | | | K2CO3 | K2CO3 | K2CO3 | K2CO3 |
| | Added amount | (mol/mol-OH) | 5.0 | 5.0 | 5.0 | 5.0 |
| | Reaction temperature | (°C) | 100 | 100 | 100 | 100 |
| | Reaction time | (Hour) | 6.0 | 6.0 | 6.0 | 6.0+6.0 |
| | OH modification rate (introduction rate) | (% by mol) | 99 | 95 | 98 | >99 |
| | Impurity | (% by mol) | 0.4 | 0.5 | 0.4 | 0.2 (MA), 0.2 (AcOH) |

**[Table 3]**

| | | | Comparativ e Example 1 | Comparativ e Example 2 | Comparativ e Example 3 | Comparativ e Example 4 | Comparativ e Example 5 |
|---|---|---|---|---|---|---|---|
| Raw material resin | | | SA90 | SA90 | SA90 | SA90 | SA90 |
| | Molecular weight | (Mn) | 2242 | 2242 | 2242 | 2242 | 2242 |
| | Molecular weight distribution | (Mw/Mn) | 1.68 | 1.68 | 1.68 | 1.68 | 1.68 |
| | Hydroxyl value | (g/mol) | 828 | 828 | 828 | 828 | 828 |
| Reaction solvent | | | Toluene | Toluene | Toluene | Toluene | Toluene |
| (Meth)acrylic acid anhydride, acyl compound, and compound for comparison | | | MAA | MAA | MAA | MAA | MAA |
| | Added amount | (mol/mol-OH) | 1.2 | 1.2 | 1.2 | 1.2 | 1.2 |
| Catalyst | | | DMAP | Na2CO3 | Li2CO3 | CaCO3 | KHCO3 |
| | Added amount | (mol/mol-OH) | 0.2 | 5.0 | 5.0 | 5.0 | 5.0 |
| | Reaction temperature | (°C) | 100 | 100 | 100 | 100 | 100 |
| | Reaction time | (Hour) | 6.0 | 6.0 | 6.0 | 6.0 | 6.0 |
| | OH modification rate (introduction rate) | (% by mol) | 92 | 21 | 5 | 3 | 7 |
| | Impurity | (% by mol) | 137 | 0.1 | 0.1 | 0.1 | 0.2 |

**[Table 4]**

| | | | Comparative Example 6 | Comparative Example 7 | Comparative Example 8 | Comparative Example 9 |
|---|---|---|---|---|---|---|
| Raw material resin | | | SA90 | SA90 | SA90 | SA90 |
| | Molecular weight | (Mn) | 2242 | 2242 | 2242 | 2242 |
| | Molecular weight distribution | (Mw/Mn) | 1.68 | 1.68 | 1.68 | 1.68 |
| | Hydroxyl value | (g/mol) | 828 | 828 | 828 | 828 |
| Reaction solvent | | | Toluene | Toluene | p-Xylene | p-Xylene |
| (Meth)acrylic acid anhydride, acyl compound, and compound for comparison | | | MAA | MAC | MA | MA |
| | Added amount | (mol/mol-OH) | 1.2 | 1.2 | 2.0 | 5.0 |
| Catalyst | | | KOH | K2CO3 | B(OH)3/ H₂SO₄ | PTSA-H₂O |
| | Added amount | (mol/mol-OH) | 5.0 | 5.0 | 0.2 | 0.1 |
| | Reaction temperature | (°C) | 100 | 100 | 130 | 130 |
| | Reaction time | (Hour) | 6.0 | 6.0 | 24 | 24 |
| | OH modification rate (introduction rate) | (% by mol) | N.D. | 46 | 4 | 6 |
| | Impurity | (% by mol) | 0.4 | 0.1 | 141 | 379 |

**[Table 5]**

| | | | Comparative Example 10 | Comparative Example 11 | Comparative Example 12 | Comparative Example 13 |
|---|---|---|---|---|---|---|
| Raw material resin | | | SA90 | SA90 | SA90 | SA90 |
| | Molecular weight | (Mn) | 2242 | 2242 | 2242 | 2242 |
| | Molecular weight distribution | (Mw/Mn) | 1.68 | 1.68 | 1.68 | 1.68 |
| | Hydroxyl value | (g/mol) | 828 | 828 | 828 | 828 |
| Reaction solvent | | | p-Xylene | DMAC | Toluene | Toluene |
| (Meth)acrylic acid anhydride, acyl compound, and compound for comparison | | | MA | MAA | MAA | MAA |
| | Added amount | (mol/mol-OH) | 5.0 | 1.2 | 1.2 | 1.2 |
| Catalyst | | | Amberlist15 | AcONa | AcONa | AcONa |
| | Added amount | (mol/mol-OH) | 10% by mass | 0.2 | 0.2 | 5.0 |
| | Reaction temperature | (°C) | 130 | 100 | 100 | 100 |
| | Reaction time | (Hour) | 24 | 6.0 | 6.0 | 6.0 |
| | OH modification rate (introduction rate) | (% by mol) | 6 | 72 | 9 | 10 |
| | Impurity | (% by mol) | 308 | 201 | 20 | 28 |

In Table 1 to Table 5, the "100 - OH modification rate" corresponds to the "proportion of a polymer having the structure represented by formula (1) wherein X is a hydrogen atom (% by mol)".

N.D. indicates being not detected.

The unit of the hydroxyl value (g/mol) indicates the mass of the polymer (raw material resin) per 1 mol of the hydroxy groups.

mol/mol-OH indicates the added amount (mol) with respect to per 1 mol of the hydroxy groups of the raw material resin (polymer having the structure represented by formula (1)).

### Example 11

The (meth)acrylic acid ester compound obtained in Example 10 (OPE-2MA) was thermally cured to produce a resin.

Specifically, 1 part by mass of PERBUTYL(R) P was added to OPE-2MA obtained in Example 10, the temperature was raised at a rate of 3°C/minute, and the mixture was vacuum heat-pressed at a pressure of 1.92 MPa and 200°C for 2 hours to obtain a cured product having a thickness of 1.2 mm.

The obtained resin was measured for the dielectric constant, the dielectric loss tangent, and the glass transition temperature.

### <Measurement of glass transition temperature>

The obtained cured product cut to a width of 5 mm and a length of 40 mm was measured for the dynamic viscoelasticity, the glass transition temperature of the cured product was defined as the peak temperature of the obtained dynamic elastic modulus. The unit was expressed in °C.
Used apparatus: DMS6100, EXSTAR6000 manufactured by Seiko Instruments Inc.
Rate of temperature rise: 5°C /min
Frequency: Sine wave, 10 Hz

### <Measurement of dielectric properties>

The obtained cured product cut to a thickness of 1.2 mm, a width of 0.8 mm, and a length of 100 mm was measured for the dielectric constant and the dielectric loss tangent at 10 GHz by cavity resonance perturbation.
Used apparatus: 8722ESNetworkAnalyzer manufactured by Agilent Technologies Japan, Ltd.

The obtained cured product had a dielectric constant (Dₖ) of 2.461 (10 GHz), a dielectric loss tangent (Df) of 0.00423 (10 GHz), and a glass transition temperature (T_{g}) of 155°C (10Hz). The physical properties of the thermosetting resin of OPE-2MA (Dₖ, D_{f}, T_{g}) were performance values equivalent to those of the cured products of common polyphenylene ethers having methacrylic groups at both ends.

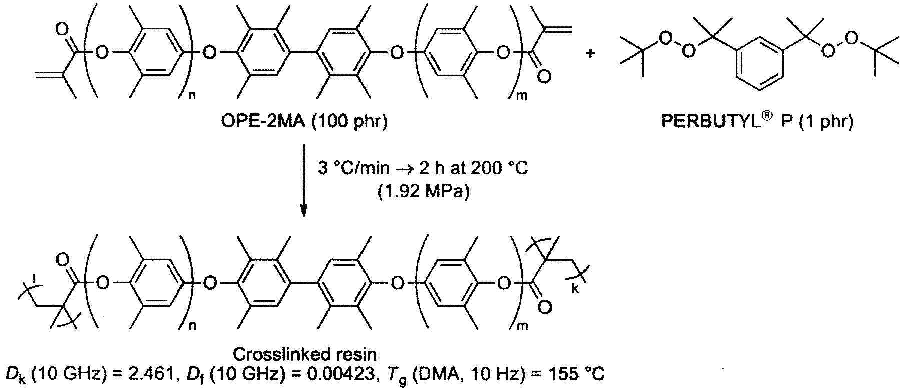

## Claims

1. A method for producing a (meth)acrylic acid ester compound, comprising: reacting a polymer having a structure represented by formula (1) with (meth)acrylic acid anhydride in the presence of at least one of potassium carbonate, rubidium carbonate, and cesium carbonate, 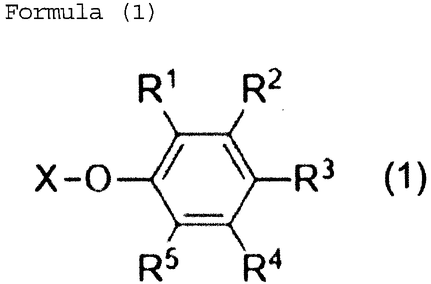 wherein R¹, R², R³, R⁴, and R⁵ are each independently selected from a hydrogen atom and an alkyl group, and at least one of R¹, R², R³, R⁴, and R⁵ is selected from a single bond, -O-*,-S-*, -S(= O)-*, - S(=O)₂-*, and an alkylene group-*, where * is an attachment position to any other site; and X is a hydrogen atom, at least some of which react with (meth)acrylic acid anhydride to be (meth)acrylic groups.

2. The method for producing a (meth)acrylic acid ester compound according to claim 1, wherein a proportion of a polymer having the structure represented by formula (1) wherein X is a hydrogen atom, after reaction with (meth)acrylic acid anhydride, in the polymer having the structure represented by formula (1) is 10% by mol or less.

3. The method for producing a (meth)acrylic acid ester compound according to claim 1 or 2, wherein the polymer having the structure represented by formula (1) is further reacted with an acyl compound, and some of X after the reaction are acyl groups.

4. The method for producing a (meth)acrylic acid ester compound according to any one of claims 1 to 3, wherein a number average molecular weight of the polymer having the structure represented by formula (1) is 1,000 to 10,000.

5. The method for producing a (meth)acrylic acid ester compound according to any one of claims 1 to 4, wherein a hydroxyl value, which is a mass of the polymer having the structure represented by formula (1) per 1 mol of hydroxy groups of the polymer, is 100 to 5,000 g/mol.

6. The method for producing a (meth)acrylic acid ester compound according to any one of claims 1 to 5, wherein a total of 1.0 to 10.0 mol of at least one of potassium carbonate, rubidium carbonate, and cesium carbonate, and 1.0 to 10.0 mol of (meth)acrylic acid anhydride are used per 1 mol of the hydroxy groups of the polymer having the structure represented by formula (1) .

7. The method for producing a (meth)acrylic acid ester compound according to any one of claims 1 to 6, wherein the polymer having the structure represented by formula (1) comprises a polymer represented by formula (2) or formula (3) 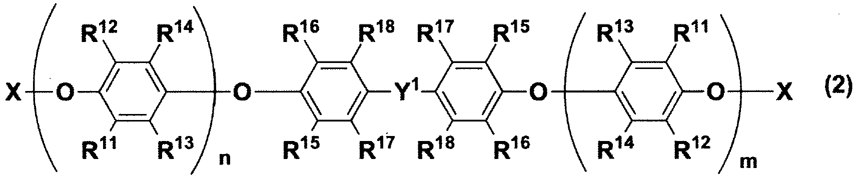 wherein R¹¹ to R¹⁸ are each independently selected from a hydrogen atom and an alkyl group; Y¹ is a single bond, -O-, -S-, -S(=O)-, - S(=O)₂-, -C(=O)-, or -C(R⁹)(R¹⁰)-, where R⁹ and R¹⁰ are each independently a hydrogen atom, an alkyl group, an alkynyl group, a hydroxy group, an amino group, an aryl group, or a heterocyclic group, and R⁹ and R¹⁰ may be bound to each other to form a ring structure; X is a hydrogen atom, at least some of which react with (meth)acrylic acid anhydride to be (meth)acrylic groups; and n is an integer of 0 or more, m is an integer of 0 or more, and m + n is an integer of 1 or more, 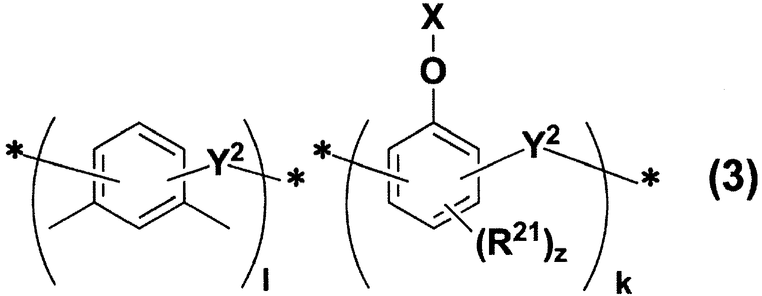 wherein R²¹ is an alkyl group, a hydroxy group, or an aryl group; Y² is -CH₂-, -CH₂O-, or -CH₂OCH₂-; X is a hydrogen atom, at least some of which react with (meth)acrylic acid anhydride to be (meth)acrylic groups; 1 is an integer of 1 or more; k is an integer of 2 or more; z is an integer of 0 to 3; and * is an attachment position to any other constituent unit or an end group.

8. The method for producing a (meth)acrylic acid ester compound according to any one of claims 1 to 7, wherein the reaction is performed in the presence of at least one of an aromatic hydrocarbon solvent and an ether solvent.
